(19) Europäisches Patentamt
European Patent Office
Office européen des brevets

(11) Veröffentlichungsnummer: **0 000 112**
**B1**

# (12) EUROPÄISCHE PATENTSCHRIFT

(45) Veröffentlichungstag der Patentschrift: **09.09.81**

(21) Anmeldenummer: **78100044.3**

(22) Anmeldetag: **01.06.78**

(51) Int. Cl.³: **C 07 D 249/08,**
A 01 N 43/64

## (54) Triazolsubstituierte Schwefelverbindungen, Verfahren zu ihrer Herstellung sowie ihre Verwendung als Fungizide.

(30) Priorität: **01.06.77 DE 2724684**

(43) Veröffentlichungstag der Anmeldung:
**20.12.78 Patentblatt 78/1**

(45) Bekanntmachung des Hinweises auf die Patenterteilung:
**09.09.81 Patentblatt 81/36**

(84) Benannte Vertragsstaaten:
**BE CH DE FR GB LU NL SE**

(56) Entgegenhaltungen:
**NL - A - 77 10992**

(73) Patentinhaber: **BASF Aktiengesellschaft**
**Carl-Bosch-Strasse 38**
**D-6700 Ludwigshafen (DE)**

(72) Erfinder: **Pommer, Ernst-Heinrich, Dr.**
**Berliner Platz 7**
**D-6703 Limburgerhof (DE)**
Erfinder: **Linhart, Friedrich, Dr.**
**Leisberg 61**
**D-6900 Heidelberg (DE)**
Erfinder: **Buschmann, Ernst, Dr.**
**In der Froschlache 7**
**D-6700 Ludwigshafen (DE)**
Erfinder: **Zeeh, Bernd, Dr.**
**Thorwaldsenstrasse 5**
**D-6700 Ludwigshafen (DE)**
Erfinder: **Hartleben, York, Dr.**
**Kleiner Ring 7**
**D-2081 Heist (DE)**
Erfinder: **Gutsche, Klaus, Dr.**
**Ehmschenkamp 5**
**D-2084 Rellingen (DE)**

EP 0 000 112 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Triazolsubstituierte Schwefelverbindungen Verfahren zu ihrer Herstellung sowie ihre Verwendung als Fungizide

Die Erfindung betrifft wertvolle triazolsubstituierte Schwefelverbindungen, ihre Salze und Metallkomplexsalze mit guter fungizider Wirkung sowie Verfahren zu ihrer Herstellung und Fungizide, die diese Verbindungen als Wirkstoffe enthalten.

Es ist bekannt, imidazolsubstituierte Schwefelverbindungen als Fungizide zur Bekämpfung pflanzenpathogener Pilze zu verwenden (DE—OS 25 41 833). Sie wirken jedoch häufig nur gegen einen bestimmten Pilz oder nur gegen eine bestimmte Pilzklasse, oder verursachen Schäden an den Kulturpflanzen oder bewirken bei der Verwendung als Beizmittel Keimverzögerungen und Auflaufschäden, so daß ihrer allgemeinen und breiten Anwendung enge Grenzen gesetzt sind.

Es wurde gefunden, daß triazolsubstituierte Schwefelverbindungen der allgemeinen Formel I

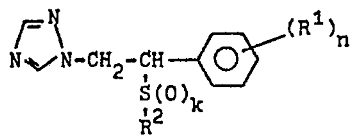

(I)

in welcher
$R^1$ Wasserstoff, Fluor, Chlor, Brom, einen Alkylrest mit 1 bis 5 Kohlenstoffatomen, eine Trifluormethyl-, Phenyl- oder Alkoxygruppe mit 1 bis 3 Kohlenstoffatomen,
n die Zahlen 1 bis 3 bedeutet, wobei für n gleich 2 oder 3 die Reste $R^1$ gleich oder verschieden sein können,
$R^2$ einen geradkettigen oder verzweigten Alkylrest mit 1 bis 14 Kohlenstoffatomen, der für den Fall des verzweigten Alkylrestes mehr als 2 Kohlenstoffatome enthält, einen Phenylrest der Formel

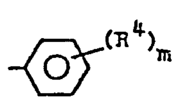 oder einen Benzylrest der Formel 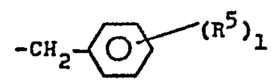

bedeutet,
$R^4$ Wasserstoff, Fluor, Chlor, Brom, einen Alkylrest mit 1 bis 5 Kohlenstoffatomen, eine Trifluormethyl-, Phenyl- oder Alkoxygruppe mit 1 bis 3 Kohlenstoffatomen,
$R^5$ Wasserstoff, Fluor, Chlor, Brom, einen Alkylrest mit 1 bis 5 Kohlenstoffatomen, eine Trifluormethyl-, Phenyl- oder Alkoxygruppe mit 1 bis 3 Kohlenstoffatomen,
m die Zahlen 1 bis 3 bedeutet, wobei für m gleich 2 oder 3 die Reste $R^4$ gleich oder verschieden sein können,
l die Zahlen 1 bis 3 bedeutet, wobei für 1 gleich 2 oder 3 die Reste $R^5$ gleich oder verschieden sein können und
k die Zahlen 0, 1 oder 2 bedeutet,
und ihre Salze und Metallkomplexsalze eine sehr gute fungizide Wirkung besitzen, keine mit den bekannten Wirkstoffen vergleichbaren Schädigungen an Kulturpflanzen hervorrufen und in ihrer ausgezeichneten Wirksamkeit gegen ein breites Spektrum von Pilzen den bekannten Wirkstoffen überlegen sind, wobei ihre gleichzeitige Wirksamkeit sowohl gegen Pilze aus der Klasse der Ascomyceten als auch gegen solche aus der Klasse der Basidiomyceten hervorzuheben ist.

Die Salze haben die allgemeine Formel II

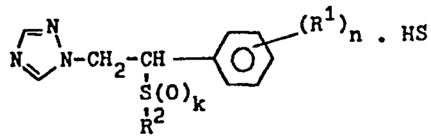

(II)

in der $R^1$, $R^2$, n und k die oben angegebenen Bedeutungen haben und HS eine beliebige organische oder anorganische Säure bedeutet, die jedoch bei den verwendeten Aufwandmengen nicht phytotoxisch sein darf und in der Lage sein muß, mit den Verbindungen der allgemeinen Formel I Salze zu bilden. Als Säuren eignen sich besonders Schwefelsäure, Salpetersäure, Salzsäure, Bromwasserstoffsäure, Jodwasserstoffsäure, Phosphorsäure, Trichloressigsäure, Dichlorpropionsäure, Oxalsäure, Toluolsulfonsäure und Dodecylbenzolsulfonsäure.

Die Verbindungen der allgemeinen Formel II können durch einfache Addition der Säuren HS an die Verbindungen der allgemeinen Formel I, gegebenenfalls in einem Lösungsmittel, hergestellt werden.

Die Metallkomplexsalze haben die allgemeine Formel IIa

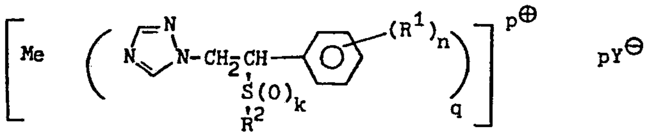

(IIa)

in der $R^1$, $R^2$, n und k die oben angegebenen Bedeutungen haben, p und q die Zahlen 1 bis 4 bedeuten, $Y^{\ominus}$ ein Äquivalent eines Anions einer anorganischen oder organischen Säure bedeutet und Me ein Metall aus der I., II. und IV. bis VIII. Nebengruppe und aus de II. und IV. Hauptgruppe des periodischen Systems darstellen kann.

Solche Metalle sind beispielsweise: Kupfer, Eisen, Zink und Zinn.

Die Metallkomplexsalze können durch Umsetzung eines Metallsalzes mit einer Verbindung der allgemeinen Formel I in einem Lösungsmittel hergestellt werden.

Die Verbindungen der allgemeinen Formel I lassen sich für den Fall $k = 0$ durch Umsetzung von 2-Halogenäthyl-1,2,4-triazolen der allgemeinen Formel III

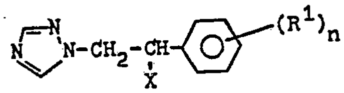

(III)

in welcher $R^1$ und n die oben angegebene Bedeutung besitzen und X Chlor, Brom oder Jod bedeutet, mit einem Mercaptid der allgemeinen Formel IV

$$^{\ominus}S—R^2 \qquad \text{(IV)}$$

in welcher $R^2$ die oben angegebene Bedeutung besitzt, in einem Lösungsmittel herstellen. Das Mercaptid wird vor oder während der Umsetzung aus dem zugehörigen Mercaptan durch Reaktion mit einer Base hergestellt.

Die Verbindungen der allgemeinen Formel III sind bekannt aus der DE—OS 25 47 954.

Die Verbindungen der allgemeinen Formel I, in denen k die Zahlen 1 oder 2 darstellt, werden aus Verbindungen der allgemeinen Formel I, in denen k die Zahl 0 bedeutet, durch Oxidation mit einem sauerstoffabgebenden Reagens nach den üblichen allgemein bekannten Verfahren der Oxidation von Sulfiden zu Sulfoxiden und Sulfonen hergestellt. Die Verbindungen der allgemeinen Formel I, bei denen $k = 2$ bedeutet, lassen isch auch durch Oxidation der Verbindungen der allgemeinen Formel I, bei denen $k = 1$ bedeutet, nach den üblichen Methoden herstellen.

In Abhängigkeit von den Bedingungen, unter denen die Reaktionen durchgeführt werden, fallen die erfindungsgemäßen fungiziden Verbindungen entweder in Form der freien Basen oder ihrer Salze an. Die Salze können in der üblichen Weise in die freien Basen umgewandelt werden, z.B. durch Umsetzung mit Alkali wie Natrium- oder Kaliumhydroxid, Natrium- oder Kaliumcarbonat, Ammoniak oder ähnlichen Alkalien. Die Verbindungen in Basenform können durch Umsetzung mit geeigneten Säuren, z.B. anorganischen Säuren, wie Salzsäure, Bromwasserstoffsäure, Jodwasserstoffsäure, Schwefelsäure, Salpetersäure, Phosphorsäure, organischen Säuren, wie Trichloressigsäure, Dichlorpropionsäure, Oxalsäure, Toluolsulfonsäure oder höhere Alkylbenzolsulfonsäuren wie Dodecylbenzolsulfonsäure in die anwendungstechnisch wertvollen Salze umgewandelt werden.

Einige charakteristische Beispiele sollen das Herstellungsverfahren erläutern.

Im folgenden verstehen sich alle Teile als Gewichtsteile und alle Prozente als Gewichtsprozente.

## Beispiel 1

21 Teile 4-Chlorthiophenol, 400 Teile Aceton, 20,2 Teile 1 - [2 - (3 - Bromphenyl) - 2 - chloräthyl - (1)] - 1,2,4 - triazol und 22 Teile Kaliumcarbonat werden unter gutem Rühren 8 Stunden zum Sieden erhitzt. Man engt ein und verrührt den Rückstand mit 200 Teilen Wasser, extrahiert 2 mal mit 150 Teilen Methylenchlorid, wäscht den Extrakt 2 mal mit 50 Teilen Wasser, trocknet mit Magnesiumsulfat, filtriert und dampft ein. Das zurückbleibende Öl wird in Äthylacetat gelöst und in die Lösung Chlorwasserstoff bis zur Sättigung eingeleitet. Beim Absaugen erhält man 30 Teile 1 - [2 - (3 - Bromphenyl) - 2 - (4 - chlorphenylmercapto) - äthyl - (1)] - 1,2,4 - triazolhydrochlorid, das nach dem Umkristallisieren aus Isopropanol bei 172 bis 173°C schmilzt.

## Beispiel 2

Eine Mischung von 24 Teilen 4-Chlorthiophenol, 22 Teilen 1 - [2 - Chlor - 2 - (2,4 - dichlorphenyl) - äthyl - (1)] - 1,2,4 - triazol, 25 Teilen Kaliumcarbonat und 400 Teilen Aceton wird 12 Stunden unter Luftausschluß zum Sieden erhitzt. Anschließend wird filtriert und das Filtrat eingeengt. Nach dem Umkristallisieren aus Cyclohexan verbleiben 22 Teile 1 - [2 - (4 - Chlorphenylmercapto) - 2 - (2,4 - dichlorphenyl) - äthyl - (1)] - 1,2,4 - triazol vom Schmelzpunkt 72°C.

Beispiel 3

Zu einer gut gerührten Suspension von 3,33 Teilen Natriumhydrid in 150 Teilen Tetrahydrofuran gibt man 22,4 Teile 4-Chlorbenzylmercaptan und rührt 1/2 Stunde. Anschließend tropft man 27,5 Teile 1 - [2 - Chlor - 2 - (2,4 - dichlorphenyl) - äthyl - (1)] - 1,2,4 - triazol, gelöst in 40 Teilen Tetrahydrofuran, zu und läßt 2 Tage rühren. Danach werden 10 Teile Wasser zugetropft und die Reaktionsmischung wird eingedampft. Man verrührt den Rückstand mit 200 Teilen Wasser und ebensoviel Essigester, trennt den letzteren ab, wächst ihn mit Wasser, trocknet mit Natriumsulfat, filtriert und leitet Chlorwasserstoff bis zur Sättigung ein. Es fallen 30,6 Teile 1 - [2 - (4 - Chlorbenzylmercapto) - 2 - (2,4 - dichlorphenyl) - äthyl - (1)] - 1,2,4 - triazolhydrochlorid aus, die nach dem Umkristallisieren bei 178°C schmelzen.

Beispiel 4

3,33 Teile Natriumhydrid werden in 150 Teilen Tetrahydrofuran suspendiert und mit 12,6 Teilen Butylmercaptan zur Reaktion gebracht. Dann gibt man bei Raumtemperatur 27,5 Teile 1 - [2 - Chlor - 2 - (2,4 - dichlorophenyl) - äthyl - (1)] - 1,2,4 - triazol, die man zuvor in 50 Teilen Tetrahydrofuran gelöst hat, zu und läßt 3 Tage rühren. Anschließend tropft man 10 Teile Wasser zu, dampft ein, schlämmt mit Wasser auf und extrahiert mit Äthylacetat. In den Extrakt gast man Chlorwasserstoff ein, wobei 25,9 Teile 1 - [2 - Butylmercapto - 2 - (2,4 - dichlorphenyl) - äthyl - (1)] - 1,2,4 - triazolhydrochlorid ausfallen, die nach dem Umkristallisieren aus Äthylacetat bei 143°C schmelzen.

Beispiel 5

10 Teile 1 - [2 - (4 - Chlorphenylmercapto) - 2 - (2,4 - dichlorphenyl) - äthyl - (1)] - 1,2,4 - triazol werden in 100 Teilen Diäthyläther gelöst und mit einer gesättigten Lösung von Oxalsäure in Diäthyläther versetzt. Es fallen 9,5 Teile 1 - [2 - (4 - Chlorphenylmercapto) - 2 - (2,4 - dichlorphenyl) - äthyl - (1)] - 1,2,4 - triazoloxalat mit dem Schmelzpunkt 155°C aus.

Beispiel 6

10 Teile 1 - [2 - (4 - Chlorphenylmercapto) - 2 - (2,4 - dichlorphenyl) - äthyl - (1)] - 1,2,4 - triazol werden in 50 Teilen Eisessig gelöst und bei 10°C mit 2,95 Teilen 30 prozentigem Wasserstoffperoxid versetzt. Nach mehrtägigem Stehen bei Raumtemperatur gießt man auf 500 Teile Wasser, extrahiert die Mischung mit Methylenchlorid, wäscht letzteres mit Wasser, trocknet es mit Magnesiumsulfat und dampft ein. Man erhält 8 Teile 1 - [2 - (2 - Chlorphenylmercapto) - 2 - (2,4 - dichlorphenyl) - äthyl - (1)] - 1,2,4 - triazoloxid, die nach dem Umkristallisieren aus Tetrachlormethan bei 127°C schmelzen.

Beispiel 7

10 Teile 1 - [2 - (4 - Chlorphenylmercapto) - 2 - (2,4 - dichlorphenyl) - äthyl - (1)] - 1,2,4 - triazol werden in einer Mischung aus 50 Teilen Eisessig und 50 Teilen Acetanhydrid gelöst und mit 9 Teilen 30%igem Wasserstoffperoxid versetzt. Man erwärmt 1 Tag auf 80°C, gießt dann auf 300 Teile Wasser und saugt die ausfallenden Kristalle ab. Man erhält 8,4 Teile 1 - [2 - (4 - Chlorphenylmercapto) - 2 - (2,4 - dichlorphenyl) - äthyl - (1)] - 1,2,4 - triazoldioxid, die nach dem Reinigen bei 145°C schmelzen.

Die folgende Tabelle zeigt ein Auswahl der erfindungsgemäßen biologisch aktiven Schwefelverbindungen der allgemeinen Formel I bz. II, welche auf die in den Beispielen angegebene Art hergestellt wurden:

Tabelle 1

| Nr. | $(R^1)_n$ | $R^2$ | k | HS | Fp.(°C) |
|---|---|---|---|---|---|
| 1 | 3-Br | 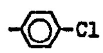 | 0 | HCl | 173 |
| 2 | 3-Br | 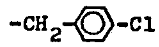 | 0 | $1/2H_2C_2O_4$ | 161 |
| 3 | 4-Cl | 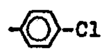 | 0 | — | 94—95 |
| 4 | 4-Cl | 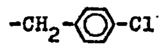 | 0 | $HNO_3$ | 109—110 |
| 5 | 4-Br | 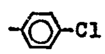 | 0 | — | 120—121 |
| 6 | 4-Br | 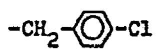 | 0 | $HNO_3$ | 119 |
| 7 | 2,4-Cl | 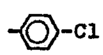 | 0 | — | 72 |
| 8 | 2,4-Cl | 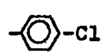 | 0 | $HNO_3$ | 160—161 |
| 9 | 2,4-Cl | 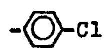 | 0 | $1/2H_2C_2O_4$ | 155 |
| 10 | 2,4-Cl | 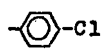 | 1 | — | 127 |
| 11 | 2,4-Cl | 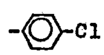 | 2 | — | 145 |
| 12 | 2,4-Cl | 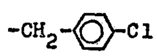 | 0 | — | 148—149 |
| 13 | 2,4-Cl | 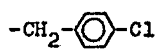 | 0 | HCl | 178 |
| 14 | 2,4-Cl | 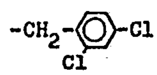 | 0 | HCl | 215 |
| 15 | $4-C(CH_3)_3$ | 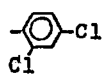 | 0 | $1/2H_2C_2O_4$ | 128—130 |
| 16 | 2,4-Cl | $—(CH_2)_3—CH_3$ | 0 | HCl | 143 |
| 17 | 2,4-Cl | $—(CH_2)_{11}—CH_3$ | 0 | HCl | 79 |
| 18 | 2,4-Cl | 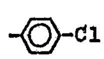 | 0 | $1/2H_2C_2O_4$ | 139 |
| 19 | 2,4-Cl | $—(CH_2)_3—CH_3$ | 0 | — | — (Öl) |

Allgemeine Formel IIa

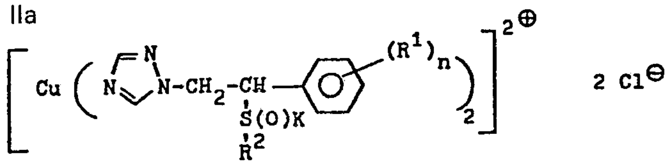

| $(R^1)_n$ | $R^2$ | k | Fp.(°C) |
|---|---|---|---|
| 2,4-Cl | 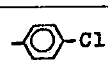 | 0 | 196 |
| 2,4-Cl | 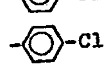 | 1 | — (Zers.) |
| 2,4-Cl | 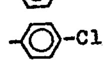 | 2 | — (Zers.) |
| 2,4-Cl | 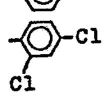 | 0 | 138—140 (Zers.) |
| 2,4-Cl | 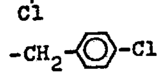 | 0 | 180 |
| 2,4-Cl | $—(CH_2)_3—CH_3$ | 0 | 133 |
| 3-Br | 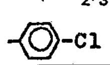 | 0 | 166 |

Die erfindungsgemäßen triazolsubstituierten Schwefelverbindungen und ihre Salze und Metallkomplexsalze zeichen sich durch eine hervorragende Wirksamkeit gegen ein breites Spektrum von pflanzenpathogenen Pilzen aus. Sie sind zum Teil systemisch wirksam und können als Blatt- und Bodenfungizide, aber auch als Beizmittel sowie als technische Fungizide eingesetzt werden.

Besonders interessant sind die fungiziden Verbindungen für die Bekämpfung einer Vielzahl von Pilzen an verschiedenen Kulturpflanzen. Unter Kulturpflanzen verstehen wir in diesem Zusammenhang insbesondere Weizen, Roggen, Gerste, Hafer, Reis, Mais, Baumwolle, Soja, Kaffee, Zuckerrohr, Obst und Zierpflanzen im Gartenbau, sowie Gemüse wie Gurken, Bohnen und Kürbisgewächse.

Die Verbindungen sind insbesondere geeignet zur Bekämpfung folgender Pflanzenkrankheiten:
Erysiphe graminis (echter Mehltau) an Getreide,
Erysiphe cichoriacearum (echter Mehltau) an Kürbisgewächsen,
Podosphaera leucotricha an Äpfeln,
Uncinula necator an Reben,
Erysiphe polygoni an Bohnen,
Sphaerotheca pannosa an Rosen,
Puccinia-Arten an Getreide,
Uromvces-Arten an Bohnen.

Die erfindungsgemäßen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls under Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage:

Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Äthanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyäthylen — Fettalkohol — Äther, Alkylsulfonate und Arylsulfonate) und Dispergiermittel, wie Lignin, Sulfitablaugen und Methylcellulose.

Die Formulierungen enthalten im allgemeinen zwischen 0,1 und 95 Gew.% Wirkstoff, vorzugsweise zwischen 0,5 und 90%.

Die Formulierungen, bzw. die daraus hergestellten gebrauchsfertigen Zubereitungen, wie Lösungen, Emulsionen, Suspensionen, Pulver, Stäube, Beizen, Pasten oder Granulate werden in bekannter Weise angewendet.

Die Aufwandmengen liegen nach Art des gewünschten Effektes zwischen 0,01 und 3, vorzugsweise jedoch zwischen 0,01 und 1 kg Wirkstoff pro Hektar.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des fungiziden Wirkungsspektrums; bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten.

Die folgende Liste von Fungiziden, mit denen die erfingungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Dithiocarbamate und deren Derivate, wie
Ferridimethyldithiocarbamat,
Zinkdimethyldithiocarbamat,
Manganäthylenbisdithiocarbamat,
Mangan-Zink-äthylendiamin-bis-dithiocarbamat,
Zinkäthylenbisdithiocarbamat,
Tetramethylthiuramdisulfide,
Ammoniak-Komplex von Zink-(N,N-äthylen-bis-dithiocarbamat) und N,N'-Polyäthylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und N,N'-Polypropylen-bis-(thiocarbamoyl)-disulfid

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Strukturen, wie
N-Trichlormethylthio-tetrahydrophthalimid,
N-Trichlormethylthio-phthalimid,
2-Heptadecyl-2-imidazolin-acetat,
2,4-Dichloro-6-(o-chloranilino)-s-triazin,
O,O-Diäthyl-phthalimidophosphonothioat,
5-Amino-1-(bis-(dimethylamino)-phosphinyl)-3-phenyl-1,2,4-triazol,
5-Äthoxy-3-trichlormethyl-1,2,4-thiadiazol,
2,3-Dicyano-1,4-dithiaanthrachinon,
2-Thio-1,3-dithio-(4,5-b)-chinoxylin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2)-benzimidazol,
5-Methyl-5-vinyl-3-(3,5-dichlorphenyl)-2,4-dioxo-1,3-oxazolidin,
N-Cyclohexyl-2,5-dimethylfuran-3-hydroxamsäuremethylester,
Piperazin-1,4-diyl-bis-(1-(2,2,2-trichlor-äthyl)-formamid),
2-(Thiazolyl-(4)-benzimidazol,
5-Butyl-2-dimethylamino-4-hydroxy-6-methyl-pyrimidin,
Bis-(p-Chlorphenyl)-3-pyridinmethanol,
1,2-Bis-(3-äthoxycarbonyl-2-thioureido)-benzol,
1,2-Bis-(3-methoxycarbonyl-2-thioureido)-benzol

und verschiedene Fungizide, wie
Dodecylguanidinacetat,
3-(2-(3,5-Dimethyl-2-oxycyclohexyl)-2-hydroxyäthyl)-glutarimid,
Hexachlorbenzol,
N-Dichlorfluormethylthio-N′,N′-dimethyl-N-phenyl-schwefelsäurediamid,
2,5-Dimethyl-furan-3-carbonsäureanilid,
2,5-Dimethyl-furan-3-carbonsäure-cyclohexylamid,
2-Methyl-benzoesäure-anilid,
2-Jod-benzoesäure-anilid,
1-(3,4-Dichloranilino)-1-formylamino-2,2,2-trichloräthan,
2,6-Dimethyl-N-tridecyl-morpholin bzw. dessen Salze,
2,6-Dimethyl-N-cyclododecyl-morpholin bzw. dessen Salze.

Für die folgenden Versuche wurden zu Vergleichszwecken die beiden folgenden bekannten Wirkstoffe verwendet

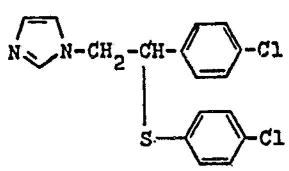

(A)

bekannt aus DT—OS 25 41 833

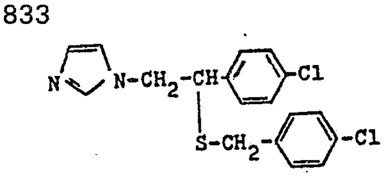

(B)

bekannt aus DT—OS 25 41 833

Beispiel 8

Wirkung gegen Gerstenmehltau

Blätter von in Töpfen gewachsenen Gerstenkeimlingen werden mit wäßrigen Emulsionen aus 80% (Gew.%) Wirkstoff und 20% Emulgiermittel besprüht und nach dem Antrocknen des Spritzbelages mit Oidien (Sporen) des Gerstenmehltaus (Erysiphe graminis var. hordei) bestäubt. Die Versuchspflanzen werden anschließend im Gewächshaus bei Temperaturen zwischen 20 und 22°C und 75 bis 80% relativer Luftfeuchtigkeit aufgestellt. Nach 10 Tagen wird das Ausmaß der Mehltaupilzentwicklung ermittelt.

| Wirkstoff Nr. | Befall der Blätter nach Spritzung mit 0,05%iger Wirkstoffbrühe |
|---|---|
| 7 | 0 |
| 9 | 0 |
| 13 | 0 |
| 8 | 1 |
| 10 | 0 |
| 11 | 0 |
| 4 | 0 |
| 3 | 2 |
| 6 | 0 |
| 5 | 2 |
| 2 | 0 |
| 1 | 0 |
| B (bekannt) | 3 |
| unbehandelt (Kontrolle) | 5 |

0 kein Befall, abgestuft bis 5 Totalbefall

## Beispiel 9

Wirkung gegen Weizenmehltau

Blätter von in Töpfen gewachsenen Weizenkeimlingen der Sorte "Jubilar" werden mit wäßrigen Emulsionen aus 80% (Gewichtsprozent) Wirkstoff und 20% Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80% relativer Luftfeuchtigkeit aufgestellt. Nach 10 Tagen wird das Ausmaß der Mehltauentwicklung ermittelt.

| | Befall der Blätter nach Spritzung mit ... %iger Wirkstoffbrühe | | |
|---|---|---|---|
| Wirkstoff Nr. | 0,05 | 0,025 | 0,012 |
| 7 | 0 | 2 | 4 |
| 9 | 0 | 3 | 3—4 |
| 3 | 0 | 2—3 | 3 |
| B (bekannt) | 3 | 4 | 5 |
| unbehandelt (Kontrolle) | 5 | | |

0 kein Befall, abgestuft bis 5 Totalbefall.

## Beispiel 10

Wirkung gegen Weizenbraunrost

Blätter von in Töpfen gewachsenen Weizensämlingen der Sorte "Caribo" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschließend mit 0,05%, 0,025 und 0,012%igen (Gew.%) wäßrigen Spritzbrühen, die 80% Wirkstoff und 20% Ligninsulfonat in der Trockensubstanz enthalten, tropfnass gespritzt. Nach dem Antrocknen des Spritzbelages werden die Versuchspflanzen im Gewächshaus bei Temperaturen zwischen 20 und 22°C und 65 bis 70% relativer Luftfeuchte aufgestellt. Nach 8 Tagen wird das Ausmaß der Rostpilzentwicklung auf den Blättern ermittelt.

| | Befall der Blätter nach Spritzung mit ... %iger Wirkstoffbrühe | | |
|---|---|---|---|
| Wirkstoff Nr. | 0,05 | 0,025 | 0,012 |
| 7 | 0 | 0 | 2 |
| 9 | 0 | 2 | 2 |
| 3 | 0 | 0 | 0 |
| A (bekannt) | 0 | 2 | 3 |
| B (bekannt) | 2 | 3 | 5 |
| unbehandelt (Kontrolle) | 5 | | |

0 kein Befall, abgestuft bis 5 Totalbefall

## Beispiel 11

Wirkung gegen Haferkronenrost

In gleicher Weise wie in Beispiel 10 angeführt, werden Blätter von in Töpfen gewachsenen Hafersämlengen der Sorte "Flämings Krone" mit Sporen des Haferkronenrostes (Puccinia coronata) bestäubt und in eine Kammer mit hoher Luftfeuchtigkeit gestellt. Die infizierten Pflanzen werden anschliesend mit 0,05%igen (Gew.%) wäßrigen Spritzbrühen, die 80% Wirkstoff und 20% Ligninsulfonat in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70% rel. Luftfeuchte aufgestellt. Nach 8 Tagen wird das Ausmaß der Rostpilzentwicklung auf den Blättern ermittelt.

| Wirkstoff Nr. | Befall der Blätter nach Spritzung mit 0,05 %iger Wirkstoffbrühe |
|---|---|
| 7 | 0 |
| 9 | 0 |
| 8 | 0 |
| 4 | 0 |
| 3 | 0 |
| B (bekannt) | 3 |
| unbehandelt (Kontrolle) | 5 |

0 kein Befall, abgestuft bis 5 Totalbefall

## Beispiel 12

Wirkung gegen Gerstenmehltau
Beizmittelanwendung

100 g-Proben Gerstensaatgut der Sorte "Asse" werden in Glasflaschen etwa 5 Minuten lang mit jeweils 300 mg (= 0,3 Gew.%) der in der Tabelle angeführten Beizmittel sorgfältig gebeizt. Danach werden jeweils 8 Körner in Töpfe eingelegt und mit Erde bedeckt. Zehn Tage nach dem Auflauf des Getreides werden die Blätter mit Oidium (Konidien) des Gerstenmehltaus (Erysiphe graminis var. hordei) bestäubt. Die Versuchspflanzen werden anschließend im Gewächshaus bei Temperaturen zwischen 20 und 22°C rel. Luftfeuchte aufgestellt. Nach weiteren 10 Tagen wird das Ausmaß der Mehltaupilzentwicklung auf den Blättern ermittelt.

| Wirkstoff Nr. | ... % Wirkstoff im Beizmittel | Ausmaß des Mehltaubefalls auf den Blättern 10 Tage nach künstlicher Infektion |
|---|---|---|
| 3 | 20 | 0 |
| 5 | 20 | 0 |
| A (bekannt) | 20 | 5 |
| B (bekannt) | 20 | 5 |
| unbehandelt (Kontrolle) | — | 5 |

0 kein Befall, abgestuft bis 5 Totalbefall

Beispiel 13

Man vermischt 90 Gewichtsteile der Verbindung 1 mit 10 Gewichtsteilen N-Methyl-$\alpha$-pyrrolidon und erhält eine Lösung, die zur Anwendung in Form kleinster Tropfen geeignet ist.

Beispiel 14

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Olsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

Beispiel 15

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 16

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 17

20 Gewichtsteile des Wirkstoffs 2 werden mit 3 Gewichtsteilen des Natriumsalzes der Diisobutyl-naphthalin-$\alpha$-sulfonsäure, 17 Gewichtsteilen des Natriumsalzes einer Ligninsulfonsäure aus einer Sulfit-Ablauge und 60 Gewichtsteilen pulverförmigen Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 18

3 Gewichtsteile der Verbindung 3 werden mit 97 Gewichtsteilen feinteiligem Kaolin innig vermischt. Man erhält auf diese Weise ein Stäubemittel, das 3 Gewichtsprozent des Wirkstoffs enthält.

Beispiel 19

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 20

40 Gewichtsteile des Wirkstoffs 1 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel 21

20 Teile des Wirkstoffs 2 werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Beispiel 22

9,58 Teile 1 - [2 - (4 - Chlorphenylmercapto) - 2 - (2,4 - dichlorphenyl) - äthyl - (1)] - 1,2,4 - triazol werden in 300 Teilen Äthanol gelöst und tropfenweise mit einer Lösung von 2,2 Teilen hydratwasserhaltigem Kupfer (II) - chlorid in 3 Teilen Wasser versetzt. Nach 20-minutigem Rühren wird abgesaugt. Man erhält 10 Teile Bis - [1 - (2 - (4 - chlorphenylmercapto - 2 - (2,4 - dichlorphenyl) - äthyl - (1)) - 1,2,4 - triazol] - Kupfer (II) - chlorid vom Schmelzpunkt 196°C.

**Patentansprüche**

1. Triazolsubstituierte Schwefelverbindungen der allgemeinen Formel I

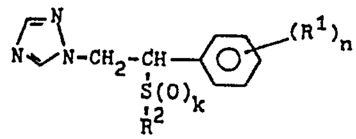

(I)

in welcher
$R^1$ Wasserstoff, Fluor, Chlor, Brom, einen Alkylrest mit 1 bis 5 Kohlenstoffatomen, eine Trifluormethyl-, Phenyl- oder Alkoxygruppe mit 1 bis 3 Kohlenstoffatomen,
n die Zahlen 1 bis 3 bedeutet, wobei für n gleich 2 oder 3 die Reste $R^1$ gleich oder verschieden sein können,
$R^2$ einen geradkettigen oder verzweigten Alkylrest mit 1 bis 14 Kohlenstoffatomen, der für den Fall des verzweitigten Alkylrestes mehr als 2 Kohlenstoffatome enthält, einen Phenylrest der Formel

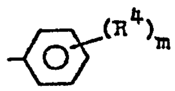 oder einen Benzylrest der Formel 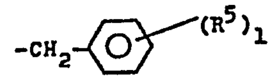

bedeutet,
$R^4$ Wasserstoff, Fluor, Chlor, Brom, einen Alkylrest mit 1 bis 5 Kohlenstoffatomen, eine Trifluormethyl-, Phenyl- oder Alkoxygruppe mit 1 bis 3 Kohlenstoffatomen,
$R^5$ Wasserstoff, Fluor, Chlor, Brom, einen Alkylrest mit 1 bis 5 Kohlenstoffatomen, eine Trifluormethyl-, Phenyl- oder Alkoxygruppe mit 1 bis 3 Kohlenstoffatomen,
m die Zahlen 1 bis 3 bedeutet, wobei für m gleich 2 oder 3 die Reste $R^4$ gleich oder verschieden sein können,
l die Zahlen 1 bis 3 bedeutet, wobei für 1 gleich 2 oder 3 die Reste $R^5$ gleich oder verschieden sein können und
k die Zahlen 0,1 oder 2 bedeutet und ihre Salze und Metallkomplexsalze.

2. Fungizid, enthaltend eine triazolsubstituierte Schwefelverbindung der allgemeinen Formel I gemäß Anspruch 1.

3. Verfahren zur Bekämpfung von Pilzen, dadurch gekennzeichnet, daß man die vor Pilzbefall zu schützenden Gegenstände behandelt mit einer triazolsubstituierten Schwefelverbindung der allgemeinen Formel I gemäß Anspruch 1.

4. 1-[2-(4-Bromphenyl)-2-(4-chlorphenylmercapto)-äthyl-(1)]-1,2,4-triazol.

5. 1-[2-(4-Bromphenyl)-2-(4-chlorbenzylmercapto)-äthyl-(1)]-1,2,4-triazol.

6. 1-[2-(3-Bromphenyl)-2-(4-chlorphenylmercapto)-äthyl-(1)]-1,2,4-triazol.

7. 1-[2-(3-Bromphenyl)-2-(4-chlorbenzylmercapto)-äthyl-(1)]-1,2,4-triazol.

8. 1-[2-(4-Chlorphenyl)-2-(4-chlorphenylmercapto)-äthyl-(1)]-1,2,4-triazol.

9. 1-[2-(4-Chlorbenzylmercapto)-2-(4-chlorphenyl)-äthyl-(1)]-1,2,4-triazol.

10. 1-[2-(4-Chlorphenylmercapto)-2-(2,4-dichlorphenyl)-äthyl-(1)]-1,2,4-triazol.

11. 1-[2-(4-Chlorphenylmercapto)-2-(2,4-dichlorphenyl)-äthyl-(1)]-1,2,4-triazoloxid.

12. 1-[2-(4-Chlorphenylmercapto)-2-(2,4-dichlorphenyl)-äthyl-(1)]-1,2,4-triazoldioxid.

13. 1-[2-(4-Chlorbenzylmercapto)-2-(2,4-dichlorphenyl)-äthyl-(1)]-1,2,4-triazoldioxid.

14. 1-[2-(4-Dichlorbenzylmercapto)-2-(2,4-dichlorphenyl)-äthyl-(1)]-1,2,4-triazol.

15. 1-[2-(4-tert.Butylphenyl)-2-(2,4-dichlorbenzylmercapto)-äthyl-(1)]-1,2,4-triazol.

16. 1-[2-Butylmercapto-2-(2,4-dichlorphenyl)-äthyl-(1)]-1,2,4-triazol.

17. 1-[2-(2,4-Dichlorphenyl)-2-dodecylmercaptoäthyl-(1)]-1,2,4-triazol.

**Revendications**

1. Composés soufrés à substituant triazole, de formule générale I

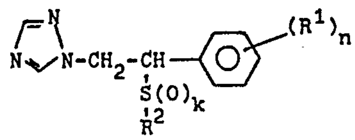

(I)

dans laquelle
$R^1$ représente hydrogène, fluor, chlore, brome, un reste alkyle en $C_1$ à $C_5$, un groupe trifluorométhyle, phényle ou alcoxy en $C_1$ à $C_3$
*n* représente un des nombres 1 à 3, sous réserve que, si *n* est égal à 2 ou 3, les restes $R^1$ peuvent être identiques ou différents,

$R^2$ représente un reste alkyle linéaire ou ramifié en $C_1$ à $C_{14}$ qui, dans le cas du reste alkyle ramifié, contient plus de deux atomes de carbone, un reste phényle de formule

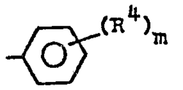 ou encore un reste benzyle de formule 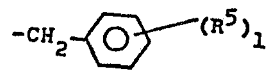

$R^4$ représente hydrogène, fluor, chlore, brome, un reste alkyle en $C_1$ à $C_5$, un groupe trifluorométhyle-, phényle- ou alcoxy en $C_1$ à $C_3$,
$R^5$ représente hydrogène, fluor, chlore, brome, un reste alkyle en $C_1$ à $C_5$, un groupe trifluorométhyle-, phényle- ou alcoxy en $C_1$ à $C_3$
*m* est un nombre de 1 à 3, sous réserve que, si *m* est égal à 2 ou 3, les restes $R^4$ peuvent être identiques ou différents,
*l* représente un nombre de 1 à 3, sous réserve que, si *l* est égal à 2 ou 3, les restes $R^5$ peuvent être identiques ou différents,
*k* représente les nombres 0, 1, 2, et les sels et sels complexes métalliques des dits composés.

2. Fongicide contenant un composé soufré substitué par un triazole, de formule générale I selon la revendication 1.

3. Procédé pour lutter contre les champignons, caractérisé par le fait que l'on traite les objets à protéger contre les dégâts des champignons avec un composé soufré, substitué par un triazole, de formule générale I selon la revendication 1.

4. 1-[2-(4-bromophényl)-2-(4-chlorophénylmercapto)-éthyl-(1)]-1,2,4-triazole.
5. 1-[2-(4-bromophényl)-2-(4-chlorobenzylmercapto)-éthyl-(1)]-1,2,4-triazole.
6. 1-[2-(3-bromophényl)-2-(4-chlorophénylmercapto)-éthyl-(1)]-1,2,4-triazole.
7. 1-[2-(3-bromophényl)-2-(4-chlorobenzylmercapto)-éthyl-(1)]-1,2,4-triazole.
8. 1-[2-(4-chlorophényl)-2-(4-chlorophénylmercapto)-éthyl-(1)]-1,2,4-triazole.
9. 1-[2-(4-chlorobenzylmercapto)-2-(4-chlorophényl)-éthyl-(1)]-1,2,4-triazole.
10. 1-[2-(4-chlorophénylmercapto)-2-(2,4-dichlorophényl)-éthyl-(1)]-1,2,4-triazole.
11. 1-[2-(4-chlorophénylmercapto)-2-(2,4-dichlorophényl)-éthyl-(1)]-1,2,4-triazoloxyde.
12. 1-[2-(4-chlorophénylmercapto)-2-(2,4-dichlorophényl)-éthyl-(1)]-1,2,4-triazoldioxyde.
13. 1-[2-(4-chlorobenzylmercapto)-2-(2,4-dichlorophényl)-éthyl-(1)]-1,2,4-triazole.
14. 1-[2-(2,4-dichlorobenzylmercapto)-2-(2,4-dichlorophényl)-éthyl-(1)]-1,2,4-triazole.
15. 1-[2-(4-tert.butylphényl)-2-(2,4-dichlorobenzylmercapto)-éthyl-(1)]-1,2,4-triazole.
16. 1-[2-butylmercapto-2-(2,4-dichlorophényl)-éthyl-(1)]-1,2,4-triazole.
17. 1-[2-(2,4-dichlorophényl)-2-dodécylmercaptoéthyl-(1)]-1,2,4-triazole.

**Claims**

1. A triazole-substituted sulfur compound of the general formula I

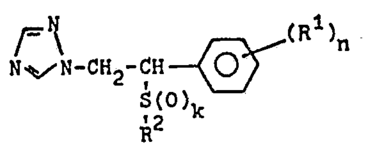 (I)

where $R^1$ denotes hydrogen, fluorine, chlorine, bromine, alkyl of 1 to 5 carbon atoms, trifluoromethyl, phenyl or alkoxy of 1 to 3 carbon atoms,
n denotes one of the integers 1, 2 and 3 ($R^1$ being identical or different when n is 2 or 3),
$R^2$ denotes a linear or branched alkyl of 1 to 14 carbon atoms, which, if it is a branched one, contains more than 2 carbon atoms, phenyl of the formula

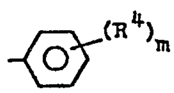 or benzyl of the formula 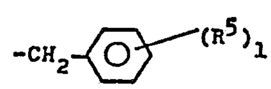

$R^4$ denotes hydrogen, fluoro, chloro, bromo, alkyl of 1 to 5 carbon atoms, trifluoromethyl, phenyl, or alkoxy of 1 to 3 carbon atoms,
$R^5$ denotes hydrogen, fluoro, chloro, bromo, alkyl of 1 to 5 carbon atoms, trifluoromethyl, phenyl, or alkoxy of 1 to 3 carbon atoms,
m denotes one of the integers 1, 2 and 3 ($R^4$ being identical or different when m is 2 or 3),
l denotes one of the integers 1, 2 and 3 ($R^5$ being identical or different when 1 is 2 or 3), and
k denotes one of the integers 0, 1 and 2, and its salts and metal complex salts.

2. A fungicide containing a triazole-substituted sulfur compound of the general formula I as claimed in claim 1.

3. A process for combating fungi, characterized in that the objects to be protected against fungus attack are treated with a triazole-substituted sulfur compound of the general formula I as claimed in claim 1.

4. 1-[2-(4-Bromophenyl)-2-(4-chlorophenylmercapto)-ethyl-(1)]-1,2,4-triazole.

5. 1-[2-(4-Bromophenyl)-2-(4-chlorobenzylmercapto)-ethyl-(1)]-1,2,4-triazole.

6. 1-[2-(3-Bromophenyl)-2-(4-chlorophenylmercapto)-ethyl-(1)]-1,2,4-triazole.

7. 1-[2-(3-Bromophenyl)-2-(4-chlorobenzylmercapto)-ethyl-(1)]-1,2,4-triazole.

8. 1-[2-(4-Chlorophenyl)-2-(4-chlorophenylmercapto)-ethyl-(1)]-1,2,4-triazole.

9. 1-[2-(4-Chlorobenzylmercapto)-2-(4-chlorophenyl)-ethyl-(1)]-1,2,4-triazole.

10. 1-[2-(4-Chlorophenylmercapto)-2-(2,4-dichlorophenyl)-ethyl-(1)]-1,2,4-triazole.

11. 1-[2-(4-Chlorophenylmercapto)-2-(2,4-dichlorophenyl)-ethyl-(1)]-1,2,4-triazole oxide.

12. 1-[2-(4-Chlorophenylmercapto)-2-(2,4-dichlorophenyl)-ethyl-(1)]-1,2,4-triazole dioxide.

13. 1-[2-(4-Chlorobenzylmercapto)-2,4-dichlorophenyl)-ethyl-(1)]-1,2,4-triazole.

14. 1-[2-(2,4-Dichlorobenzylmercapto)-2-(2,4-dichlorophenyl)-ethyl-(1)]-1,2,4-triazole.

15. 1-[2-(4-tert-Butylphenyl)-2-(2,4-dichlorobenzylmercapto)-ethyl-(1)]-1,2,4-triazole.

16. 1-[2-Butylmercapto-2-(2,4-dichlorophenyl)-ethyl-(1)]-1,2,4-triazole.

17. 1-[2-(2,4-Dichlorophenyl)-2-dodecylmercaptoethyl-(1)]-1,2,4-triazole.